# EUROPEAN PATENT SPECIFICATION

(11) **EP 2 368 119 B1**
(45) Date of publication and mention of the grant of the patent: **23.04.2014**
(21) Application number: 09760061.3
(22) Date of filing: 20.11.2009
(51) Int. Cl.: G01N 33/68

(54) **METHOD FOR DETERMINING THE RISK OF PREECLAMPSIA USING PIGF-2 AND PIGF-3 MARKERS**
VERFAHREN ZUR BESTIMMUNG DES PRÄEKLAMPSIERISIKOS UNTER VERWENDUNG VON PIGF-2- UND PIGF-3-MARKERN
PROCÉDÉ POUR DÉTERMINER LE RISQUE D ÉCLAMPSIE UTILISANT LES MARQUEURS PIGF-2 ET PIGF-3

(30) Priority: 20.11.2008 US 116366 P
(43) Date of publication of application: 28.09.2011
(73) Proprietor: PerkinElmer Health Sciences, Inc., Waltham, MA 02451 (US); NTD Laboratories, Inc., Melville, New York 11747 (US); WALLAC OY, 20101 Turku (FI)
(72) Inventor: AHOLA, Tarja, FI-20100 Turku (FI); FRANG, Heini, FI-21420 Lieto (FI); KORPIMÄKI, Teemu, FI-20100 Turku (FI); HURSKAINEN, Pertti, FI-20760 Piispanristi (FI); BOBROW, Mark N., Waltham Massachusetts 02451 (US); CARMICHAEL, Jonathan B., Mastic New York 11950 (US)
(74) Representative: Berggren Oy Ab
(86) International application number: PCT/US2009/065355
(87) International publication number: WO 2010/059952

(56) References cited:
- DE-A1-102005 022 047
- US-A1- 2007 111 326
- MOORE SIMAS ET AL: "Angiogenic factors for the prediction of preeclampsia in high-risk women" AMERICAN JOURNAL OF OBSTETRICS & GYNECOLOGY, MOSBY, ST LOUIS, MO, US, vol. 197, no. 3, 1 September 2007 (2007-09-01), pages 244.e1-244.e8, XP022254113 ISSN: 0002-9378
- AKOLEKAR R ET AL: "Maternal serum placental growth factor at 11 + 0 to 13 + 6 weeks of gestation in the prediction of pre-eclampsia." ULTRASOUND IN OBSTETRICS & GYNECOLOGY : THE OFFICIAL JOURNAL OF THE INTERNATIONAL SOCIETY OF ULTRASOUND IN OBSTETRICS AND GYNECOLOGY NOV 2008, vol. 32, no. 6, November 2008 (2008-11), pages 732-739, XP002571052 ISSN: 1469-0705 cited in the application

### SEQUENCE LISTING

<110> wallac oy, PerkinElmer Health science, Inc., NTD Laboratories, Inc.
<120> Method for determining the risk of pre-eclampsia
<130> BP201565
<150> Us61/116, 366
   <151> 2008-11-20
<160> 9
<170> PatentIn version 3.3
<210> 1
   <211> 242
   <212> PRT
   <213> Homo sapiens
<400> 1
   Met Pro Val Met Arg Leu Phe Pro Cys Phe Leu Gln Leu Leu Ala Gly
   1 5 10 15
   Leu Ala Leu Pro Ala Val Pro Pro Gln Gln Trp Ala Leu Ser Ala Gly
   20 25 30
   Asn Gly Ser Ser Glu Val Glu Val Val Pro Phe Gln Glu Val Trp Gly
   35 40 45
   Arg Ser Tyr Cys Arg Ala Leu Glu Arg Leu Val Asp Val Val Ser Glu
   50 55 60
   Tyr Pro Ser Glu Val Glu His Met Phe Ser Pro Ser Cys Val Ser Leu
   65 70 75 80
   Leu Arg Cys Thr Gly Cys Cys Gly Asp Glu Asn Leu His Cys Val Pro
   85 90 95
   Val Glu Thr Ala Asn Val Thr Met Gln Leu Leu Lys Ile Arg Ser Gly
   100 105 110
   Asp Arg Pro Ser Tyr Val Glu Leu Thr Phe Ser Gln His Val Arg Cys
   115 120 125
   Glu Cys Arg His Ser Pro Gly Arg Gln Ser Pro Asp Met Pro Gly Asp
   130 135 140
   Phe Arg Ala Asp Ala Pro Ser Phe Leu Pro Pro Arg Arg Ser Leu Pro
   145 150 155 160
   Met Leu Phe Arg Met Glu Trp Gly Cys Ala Leu Thr Gly Ser Gln Ser
   165 170 175
   Ala Val Trp Pro Ser Ser Pro Val Pro Glu Glu Ile Pro Arg Met His
   180 185 190
   Pro Gly Arg Asn Gly Lys Lys Gln Gln Arg Lys Pro Leu Arg Glu Lys
   195 200 205
   Met Lys Pro Glu Arg Arg Arg Pro Lys Gly Arg Gly Lys Arg Arg Arg
   210 215 220
   Glu Lys Gln Arg Pro Thr Asp Cys His Leu Cys Gly Asp Ala Val Pro
   225 230 235 240
   Arg Arg
<210> 2
   <211> 131
   <212> PRT
   <213> Homo sapiens
<400> 2
   Leu Pro Ala Val Pro Pro Gln Gln Trp Ala Leu Ser Ala Gly Asn Gly
   1 5 10 15
   Ser Ser Glu Val Glu Val Val Pro Phe Gln Glu Val Trp Gly Arg Ser
   20 25 30
   Tyr Cys Arg Ala Leu Glu Arg Leu Val Asp Val Val Ser Glu Tyr Pro
   35 40 45
   Ser Glu Val Glu His Met Phe Ser Pro Ser Cys Val Ser Leu Leu Arg
   50 55 60
   Cys Thr Gly Cys Cys Gly Asp Glu Asn Leu His Cys Val Pro Val Glu
   65 70 75 80
   Thr Ala Asn Val Thr Met Gln Leu Leu Lys Ile Arg Ser Gly Asp Arg
   85 90 95
   Pro Ser Tyr Val Glu Leu Thr Phe Ser Gln His Val Arg Cys Glu Cys
   100 105 110
   Arg Pro Leu Arg Glu Lys Met Lys Pro Glu Arg Cys Gly Asp Ala Val
   115 120 125
   Pro Arg Arg
   130
<210> 3
   <211> 152
   <212> PRT
   <213> Homo sapiens
<400> 3
   Leu Pro Ala Val Pro Pro Gln Gln Trp Ala Leu Ser Ala Gly Asn Gly
   1               5                   10                  15
   Ser Ser Glu Val Glu Val Val Pro Phe Gln Glu Val Trp Gly Arg Ser
               20                  25                  30
   Tyr Cys Arg Ala Leu Glu Arg Leu Val Asp Val Val Ser Glu Tyr Pro
           35                  40                  45
   Ser Glu Val Glu His Met Phe Ser Pro Ser Cys Val Ser Leu Leu Arg
       50                  55                  60
   Cys Thr Gly Cys Cys Gly Asp Glu Asp Leu His Cys Val Pro Val Glu
   65                  70                  75                  80
   Thr Ala Asn Val Thr Met Gln Leu Leu Lys Ile Arg Ser Gly Asp Arg
                   85                  90                  95
   Pro Ser Tyr Val Glu Leu Thr Phe Ser Gln His Val Arg Cys Glu Cys
               100                 105                 110
   Arg Pro Leu Arg Glu Lys Met Lys Pro Glu Arg Arg Arg Pro Lys Gly
           115                 120                 125
   Arg Gly Lys Arg Arg Arg Glu Lys Gln Arg Pro Thr Asp Cys His Leu
       130                 135                 140
   Cys Gly Asp Ala Val Pro Arg Arg
   145                 150
<210> 4
   <211> 203
   <212> PRT
   <213> Homo sapiens
<400> 4
   Leu Pro Ala Val Pro Pro Gln Gln Trp Ala Leu Ser Ala Gly Asn Gly
   1               5                   10                  15
   Ser Ser Glu Val Glu Val Val Pro Phe Gln Glu Val Trp Gly Arg Ser
               20                  25                  30
   Tyr Cys Arg Ala Leu Glu Arg Leu Val Asp Val Val Ser Glu Tyr Pro
           35                  40                  45
   Ser Glu Val Glu His Met Phe Ser Pro Ser Cys Val Ser Leu Leu Arg
       50                  55                  60
   Cys Thr Gly Cys Cys Gly Asp Glu Asn Leu His Cys Val Pro Val Glu
   65                  70                  75                  80
   Thr Ala Asn Val Thr Met Gln Leu Leu Lys Ile Arg Ser Gly Asp Arg
                   85                  90                  95
   Pro Ser Tyr Val Glu Leu Thr Phe Ser Gln His Val Arg Cys Glu Cys
               100                 105                 110
   Arg His Ser Pro Gly Arg Gln Ser Pro Asp Met Pro Gly Asp Phe Arg
           115                 120                 125
   Ala Asp Ala Pro Ser Phe Leu Pro Pro Arg Arg Ser Leu Pro Met Leu
       130                 135                 140
   Phe Arg Met Glu Trp Gly Cys Ala Leu Thr Gly Ser Gln Ser Ala Val
   145                 150                 155                 160
   Trp Pro Ser Ser Pro Val Pro Glu Glu Ile Pro Arg Met His Pro Gly
                   165                 170                 175
   Arg Asn Gly Lys Lys Gln Gln Arg Lys Pro Leu Arg Glu Lys Met Lys
               180                 185                 190
   Pro Glu Arg Cys Gly Asp Ala Val Pro Arg Arg
           195                 200
<210> 5
   <211> 224
   <212> PRT
   <213> Homo sapiens
<400> 5
   Leu Pro Ala Val Pro Pro Gln Gln Trp Ala Leu Ser Ala Gly Asn Gly
   1               5                   10                  15
   Ser Ser Glu Val Glu Val Val Pro Phe Gln Glu Val Trp Gly Arg Ser
               20                  25                  30
   Tyr Cys Arg Ala Leu Glu Arg Leu Val Asp Val Val Ser Glu Tyr Pro
           35                  40                  45
   Ser Glu Val Glu His Met Phe Ser Pro Ser Cys Val Ser Leu Leu Arg
       50                  55                  60
   Cys Thr Gly Cys Cys Gly Asp Glu Asn Leu His Cys Val Pro Val Glu
   65                  70                  75                  80
   Thr Ala Asn Val Thr Met Gln Leu Leu Lys Ile Arg Ser Gly Asp Arg
                   85                  90                  95
   Pro Ser Tyr Val Glu Leu Thr Phe Ser Gln His Val Arg Cys Glu Cys
   100 105 110
   Arg His Ser Pro Gly Arg Gln Ser Pro Asp Met Pro Gly Asp Phe Arg
   115 120 125
   Ala Asp Ala Pro Ser Phe Leu Pro Pro Arg Arg Ser Leu Pro Met Leu
   130 135 140
   Phe Arg Met Glu Trp Gly Cys Ala Leu Thr Gly Ser Gln Ser Ala Val
   145 150 155 160
   Trp Pro Ser Ser Pro Val Pro Glu Glu Ile Pro Arg Met His Pro Gly
   165 170 175
   Arg Asn Gly Lys Lys Gln Gln Arg Lys Pro Leu Arg Glu Lys Met Lys
   180 185 190
   Pro Glu Arg Arg Arg Pro Lys Gly Arg Gly Lys Arg Arg Arg Glu Lys
   195 200 205
   Gln Arg Pro Thr Asp Cys His Leu Cys Gly Asp Ala Val Pro Arg Arg
   210 215 220
<210> 6
   <211> 21
   <212> PRT
   <213> Homo sapiens
<400> 6
   Arg Arg Pro Lys Gly Arg Gly Lys Arg Arg Arg Glu Lys Gln Arg Pro
   1 5 10 15
   Thr Asp Cys His Leu
   20
<210> 7
   <211> 72
   <212> PRT
   <213> Homo sapiens
<400> 7
   His Ser Pro Gly Arg Gln Ser Pro Asp Met Pro Gly Asp Phe Arg Ala
   1 5 10 15
   Asp Ala Pro Ser Phe Leu Pro Pro Arg Arg Ser Leu Pro Met Leu Phe
   20 25 30
   Arg Met Glu Trp Gly Cys Ala Leu Thr Gly Ser Gln Ser Ala Val Trp
   35 40 45
   Pro Ser Ser Pro Val Pro Glu Glu Ile Pro Arg Met His Pro Gly Arg
   50 55 60
   Asn Gly Lys Lys Gln Gln Arg Lys
   65 70
<210> 8
   <211> 18
   <212> PRT
   <213> Homo sapiens
<400> 8
   His Ser Pro Gly Arg Gln Ser Pro Asp Met Pro Gly Asp Phe Arg Ala
   1 5 10 15
   Asp Ala
<210> 9
   <211> 19
   <212> PRT
   <213> Homo sapiens
<400> 9
   Pro Glu Glu Ile Pro Arg Met His Pro Gly Arg Asn Gly Lys Lys Gln
   1 5 10 15
   Gln Arg Lys

## Description

### BACKGROUND OF INVENTION

At least 126 million women give birth every year worldwide. Over 20 million of them experience a pregnancy related complication or illness. For example, hypertensive disorders such as pre-eclampsia (PE) affect more than 10% of all pregnancies and are a leading cause of maternal death. Adequate prenatal health care decreases the chances that such complications and illnesses will go unnoticed. Even so, currently no routine screens have been adopted for early detection of pre-eclampsia using maternal samples. If the development of PE, and.in particular early onset PE, could be detected earlier, better outcomes, including severity reduction and even recovery could be possible in many cases. During the pregnancy, at an early or later stage, a reliable risk assessment method for developing PE or assessment of the presence of PE would decrease the potential for negative health outcome of the pregnant woman, the baby or both.

Many biological markers present in maternal samples are currently recognized as associated with pre-eclampsia. Placental growth factor (PlGF) is currently suggested for use in assessing the risk of a pregnant woman developing PE (Akolckar et al. (2008) maternal serum placental growth factor at 11+0 to 13+6 weeks of gestation in the prediction of pre-eclampsia. Ultrasound Obstet Gynecol 32:732-739). US 2007/011132 A1 describes a method of determining the amount of sFlt-1, particularly free sFlt-1, and the amount PlGF, particularly free PlGF, in a sample. The method is used in a method of predicting risk of preeclampsia comprising comparing free PlGF to free sFlt-1. Although PlGF has received some acceptance as a reliable marker of pre-eclampsia, it is desirable to have alternative and additional markers characterized by greater specificity and predictive power.

### SUMMARY OF THE INVENTION

The description relates to a method for determining the risk of a pregnant woman developing pre-eclampsia. The method involves determining the level of one or more biochemical markers in a sample obtained from a pregnant woman, wherein at least one biochemical marker;is a PlGF isoform selected from PlGF-2 and PlGF-3; comparing the level of the at least one biochemical marker in the sample with the level of the same biochemical marker in a control sample; wherein a difference in the level of the at least one biochemical marker in the sample relative to the control sample is indicative of an increased risk of developing pre-eclampsia.

In an embodiment, the level of PIGF-2 is determined. In this case an increased level of PlGF-2 in the sample relative to the control sample is indicative of an increased risk of developing pre-eclampsia.

In another embodiment, the level of PlGF-3 is determined. In this case decreased level of PlGF-3 in the subject sample relative to the control sample is indicative of an increased risk of developing pre-eclampsia.

In various embodiments, combinations of PlGF isoforms are employed. For example, PlGF-2 and PlGF-3 can be used when determining the risk of a pregnant woman developing pre-eclampsia. Other exemplary PlGF isoform combinations include PlGF-2 and PlGF-1; PlGF-3 and PlGF-1; PlGF-1, PlGF-2 and PlGF3.

In further embodiments, ratios of biochemical markers are employed when determining the risk of a pregnant woman developing pre-eclampsia. In a specific embodiment, the ratio of PlGF-2/PlGF-3 is determined. In another embodiment, the ratio of PlGF-2/PlGF-1 is determined. In both of these examples, a difference in the ratio in the subject sample relative to the control sample is indicative of an increased risk of developing pre-eclampsia.

In additional embodiments, PlGF-2 and/or PlGF-3 can be used in combination with one or more of biochemical markers of pre-eclampsia, hypertension, placental disorders and the like. In specific embodiment, one or more biochemical markers selected from PAPP-A, PAI-1, PAI-2, PlGF-1, PlGF-4, PP13, VEGF165b and ADAM-12 are used. Other biochemical markers useful for detecting pre-eclampsia also can be used in combination with PlGF-2, PlGF-3 and both PlGF-2 and PlGF-3. As a specific example, when PlGF-1 is used in such combination, the level, in particular decreased level of P1GF-1 in the subject sample relative to the control sample is indicative of an increased risk of developing pre-eclampsia.

In another embodiment, the methods for determining the risk of a pregnant woman developing pre-eclampsia includes using one or more biophysical markers of the pregnant woman. The determined biophysical markers are compared with the same biophysical marker in a control subject, wherein an increased or decreased measure of each biophysical marker in the pregnant woman relative to the control is indicative of an increased risk of developing pre-eclampsia. In one embodiment the biophysical marker is blood pressure. In another embodiment, the biophysical marker is uterine artery pulsatility index.

The methods described herein for determining the risk of a pregnant woman developing pre-eclampsia can be practiced using a sample obtained from the woman during the first or second trimester of pregnancy. In a specific embodiment, the sample is obtained during the first trimester. In another embodiment, the sample is obtained during the second trimester. In an embodiment, one or more samples can be obtained from the woman at one or more stages of pregnancy.

Also described herein is a method for determining whether a pregnant woman has pre-eclampsia. The method involves determining the level of one or more biochemical markers in a sample obtained from a pregnant woman, wherein at least one biochemical marker is a PlGF isoform selected from PlGF-2 and PlGF-3; comparing the level of the at least one biochemical marker in the sample with the level of the same biochemical marker in a control sample; wherein a difference in the level of the at least one biochemical marker in the subject sample relative to the control sample is indicative of pre-eclampsia.

In an embodiment of this method, at least one of the following observations are made: the level of PIGF-2 in a sample obtained from a subject is increased relative to the level of PIGF-2 in the control sample; and/or the level of PIGF-3 in a sample obtained from a subject is decreased relative to the level of PIGF-3 in the control sample.

In an embodiment, a difference in ratio of PIGF-2/PIGF-1 in a sample obtained from a subject relative to the control sample is indicative of having pre-eclampsia. In another embodiment, a difference in the ratio of PIGF-2/PIGF-3 in a sample obtained from a subject relative to the control sample is indicative of having pre-eclampsia.

In an embodiment, one or more samples can be obtained from the pregnant woman during first and/or second trimester of pregnancy, such as between weeks 20- 40 of pregnancy.

Further described herein is a computer program which when executed on a computer causes the computer to perform a process for determining risk or presence of pre-eclampsia in a pregnant woman. The process can involve inputting a measurement of at least one biomarker obtained by: i) assaying a sample obtained from the pregnant woman for one or more biochemical markers, wherein at least one biochemical marker is selected from PIGF-2 and PIGF-3; ii) comparing the level of the one or more biochemical marker in the sample with the level of the same biochemical marker in a control sample, wherein a difference in level of the one or more biochemical marker in the sample relative to the control sample is indicative of pre-eclampsia, and iii) determining a quantitative estimate of pre-eclampsia risk based on the result of the comparing.

In an embodiment, the computer program can further involve use of at least one additional biochemical marker. In specific embodiments, the one or more biochemical markers can be selected from the group comprising PAPP-A, PAI-1, PAI-2, PlGF-1, VEGF165b, PlGF-4 and ADAM-12.

In a further embodiment, the computer program can involve inputting a measurement of at least one biomarker obtained by determining one or more biophysical markers of the subject; comparing the one or more biophysical markers of the subject with the same biophysical marker in a control subject, wherein an increased or decreased measure of the one or more biophysical marker in the subject relative to the control is indicative of an increased risk of developing pre-eclampsia, and determining a quantitative estimate of pre-eclampsia risk based on the result of the compared one of more biochemical marker and the compared one or more biophysical marker. In particular embodiments, the biophysical marker can be selected from blood pressure and uterine artery pulsatility index.

In an embodiment of the computer program, the process can also include determining the quantitative estimate of pre-eclampsia risk comprises determining the likelihood of pre-eclampsia using a multivariate analysis, and wherein the multivariate analysis comprises using levels of the biochemical markers and distribution parameters derived from a set of control reference data. In an embodiment, the multivariate analysis is a multivariate Gaussian analysis.

Additional described herein is computer program recording medium storing a computer program as is described above.

The invention described herein includes kits or commercial packages for assessing risk or presence of pre-eclampsia in a pregnant woman. In an embodiment, the kit provides i) at least two detectable binding partners, wherein each detectable binding partner binds specifically to an individual PlGF isoform selected from PIGF-1, PIGF-2 and PIGF-3.

In a specific embodiment, a kit provides i) a detectable binding partner that binds specifically to PIGF-2, and ii) a detectable binding partner that binds specifically to a PlGF isoform selected from PlGF-1 and PIGF-3.

In another specific embodiment, a kit provides i) a detectable binding partner that binds specifically to PIGF-3, and ii) a detectable binding partner that binds specifically to a PlGF isofonn selected from PlGF-1 and PIGF-2.

In embodiments, the detectable binding partner is an antibody or antigen-binding fragment thereof.

In a further specific embodiment, a kit provides i) at least one antibody or an antigen-binding fragment thereof, that binds specifically to a PlGF isoform selected from PIGF-2 and PIGF-3; and ii) instructions for using the antibody or antigen-binding fragment in the determination.

In an embodiment the antibody or antigen-binding fragment thereof binds to the loop 3 structure of the PlGF isoform. In a specific embodiment, the antibody or antigen-binding fragment thereof binds to the sequence between amino acids 124 to 144 in mature PIGF-2 (SEQ ID NO:6) or to the sequence between amino acids 114 to 185 in mature PIGF-3 (SEQ ID NO:7). In specific embodiments, the antibody or antigen-binding fragment thereof binds to a PlGF-3 sequence selected from HSPGRQSPDMPGDFRADA (SEQ ID NO:8) and PEEIPRMHPGRNGKKQQRK (SEQ ID NO:9).

In the following text, the invention will be further described with the aid of a detailed description and with reference to some working examples.

### BRIEF DESCRIPTION OF THE DRAWINGS

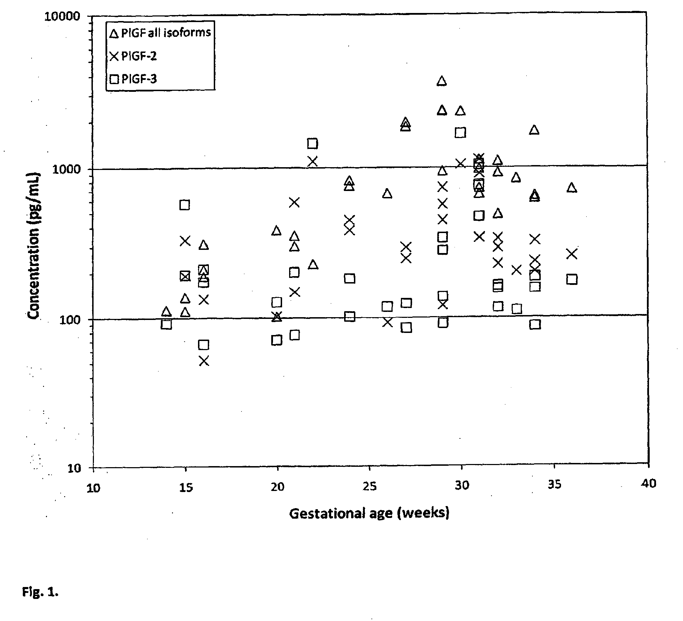

Fig. 1 shows levels of PlGF-2, PlGF-3 and combined PlGF-1, PlGF-2 and PlGF-3 in maternal serum at 13 to 37 weeks of gestation.

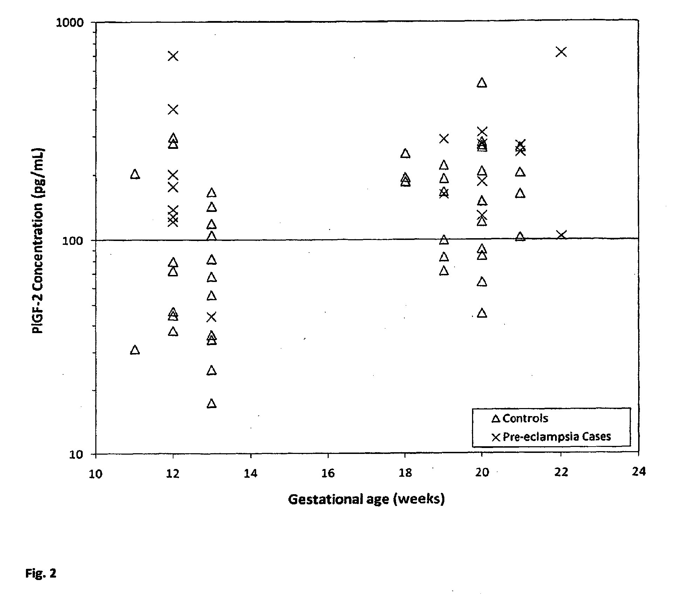

Fig. 2 describes the results of a matched case-control study in which samples taken from pregnant, non-pre-eclamptic females (controls, represented by triangles in the illustration) and samples taken from pregnant females that later on in their pregnancy developed pre-eclampsia (cases, represented by crosses in the illustration) were measured with a method that detects PlGF-2 isoform.

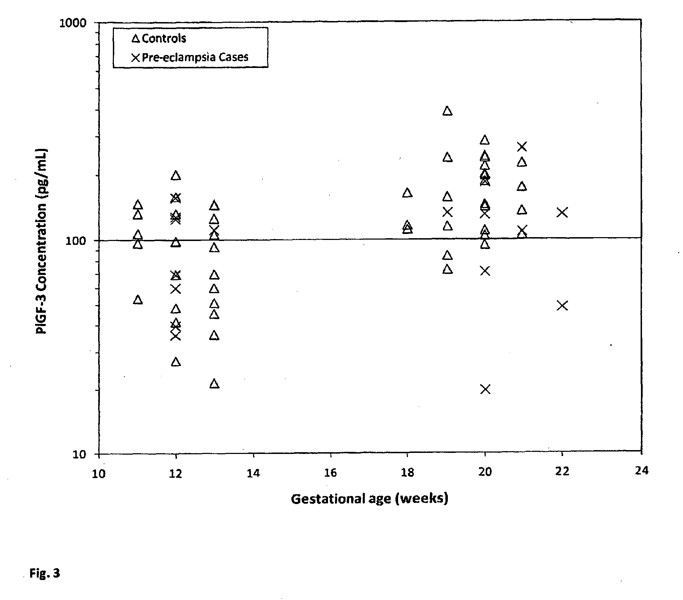

Fig. 3 describes the results of a matched case-control study in which samples taken from pregnant, non-pre-eclamptic females (controls, represented by triangles in the illustration) and samples taken from pregnant females that later on in their pregancy developed pre-eclampsia (cases, represented by crosses in the illustration) were measured with a method that detects PlGF-3 isoform.

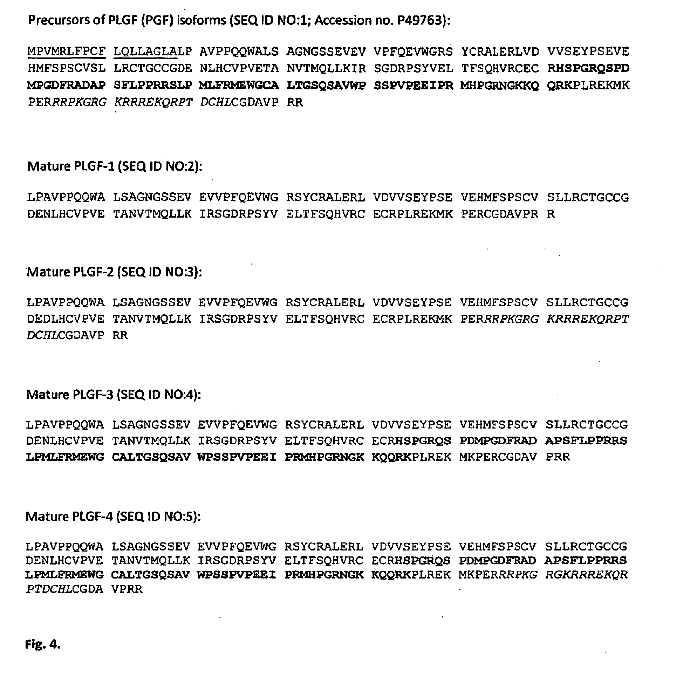

Fig. 4 describes the amino acid sequences of the PlGF Family and relatives; in the precursor amino acids 1 to 18 represent the signal sequence (underlined); in the precursor amino acids 214 to 234, in PlGF-2 amino acids 124 to 144, and in PlGF-4, amino acids 196 to 216 represent the Heparin binding domain (*italics*); in the precursor amino acids 132 to 204; in PlGF-3 amino acids 114 to 185, and in PlGF-4 amino acids 114 to 185 represent a domain of unknown function (bold).

### DETAILED DESCRIPTION OF THE INVENTION

The present invention provides antibodies and kits for assessing the risk or presence of pre-eclampsia.

The present description relates also to methods for determining the risk of a pregnant woman developing pre-eclampsia, and to methods for determining whether a pregnant woman has pre-eclampsia. Also described herein is a computer program used in these determinations.

Described herein is use of selective detection of at least one of the isoforms PlGF-2 and PlGF-3, optionally together with other biochemical markers including PlGF isoforms such as PlGF-1 and PlGF-4, and the comparison of the level of the at least one biochemical marker to a value obtained from a control sample. A difference in the level of the biochemical marker in the sample relative to control sample indicates the risk or presence of pre-eclampsia in the woman. The difference can be an increase or decrease in the level of the value, depending on the particular biomarker tested.

Previously published studies have shown that a lowered level of PlGF-1 indicates the development of pre-eclampsia. The measurement methods used in such studies appear to mainly detect PlGF-1, but in at least some cases, there is significant cross-reactivity with other PlGF isoforms (see Example 2). When the level of the isoforms PlGF-2 and PlGF-3 were selectively determined as is described herein, it was surprisingly found that the level of PlGF-2 was increased in the sample relative to the control sample. Additionally it was found that the level of PlGF-3 was decreased in the sample relative to the control sample.

As is described herein, it has now been recognized that measurement of a specific isoform of PlGF in a maternal sample can be used to better determine risk of a pregnant woman developing pre-eclampsia. Assay of a target PlGF isoform can provide more specific results in laboratory tests because background signal caused by the presence of non-target PlGF isoforms can be reduced or eliminated. The screening performance will thus be improved, as reflected by increased detection rates and lower false positive rates, relative to laboratory tests that employ non-selective PlGF detection. Also recognized is that a difference in the ratio of PlGF isoforms, for example, PlGF-2/PlGF-3 and/or PlGF-2/PlGF-1, in the subject sample relative to the control sample can be used as indicative of an increased risk of developing pre-eclampsia. Ratios of a PlGF isoform such as PlGF-2 or PlGF-3 and another biochemical marker can also be used if desired.

The selective detection of the isoforms PlGF-2 and/or PlGF-3 optionally together with another PlGF isoform, such as PlGF-1 or PIGF-4, or both, can be combined with any other suitable biochemical markers or other indicators used for assessing the risk of developing pre-eclampsia. Such biochemical markers are for example those selected from PAPP-A, PAI-1, PAI-2, PlGF-1, PlGF-4, PP13, VEGF165b and ADAM-12.

The selective detection of the isoforms PlGF-2 and/or PlGF-3 optionally together with another PlGF isoform can be combined also with any suitable biophysical markers for assessing the risk of developing pre-eclampsia. Such biochemical markers are for example blood pressure and uterine artery pulsatility index.

As used herein, the term "pre-eclampsia" means a disorder of pregnancy characterized in part by gestational hypertension and proteinuria. For previously normotensive women, PE is typically defined as gestational hypertension with proteinuria, and severe PE is typically defined as severe gestational hypertension with proteinuria. For women with chronic hypertension, superimposed PE is typically defined as the new development of proteinuria. Aspects of PE useful for making a diagnosis ofPE can be classified according to guidelines set out by various medical organizations. For example, gestational hypertension, according to guidelines of the International Society for the Study of Hypertension in Pregnancy (Davey et al., Am. J. Obstet Gynecol; 158; 892098, 1988), is described as two recordings of diastolic blood pressure of 90 mmHg or higher at least 4 h apart, and severe hypertension as pressure of at least 110 mm Hg or higher at least 4 h apart or one recording of diastolic blood pressure of at least 120 mm Hg. Proteinuria is often described as excretion of 300 mg or more in 24 h or two readings of 2+ or higher on dipstick analysis of midstream or catheter urine specimens if no 24 h collection was available. Women are classified as previously normotensive or with chronic hypertension generally before 20 weeks gestation. Pre-eclampsia is understood and shall be defined herein to encompass and reside within a spectrum of pre-eclampsia disorders, including placental insufficiency, intrauterine growth retardation, early miscarriage, preterm birth, intrauterine death and eclampsia. Although not wishing to be bound by theory, it has been proposed that intrauterine growth retardation reflects an adaptation of the pregnant woman's body to cope with the condition of pre-eclampsia, which allows the fetus to survive. Early miscarriage and preterm birth, on the other hand, can reflect adaptation of the pregnant woman's body to cope with the condition of pre-eclampsia, which allow the woman to survive. In this context, intrauterine death would be a failure of this adaptation. Thus, the methods described herein for determining risk of pre-eclampsia include, and can also be used to determine risk of pre-eclampsia disorders on the pre-eclampsia spectrum.

According to the description, a method for determining the risk of a pregnant woman developing pre-eclampsia, involves
i) determining the level of one or more biochemical markers in a sample obtained from a pregnant woman, wherein at least one biochemical marker is a PlGF isoform selected from PlGF-2 and PlGF-3;
ii) comparing the level of the at least one biochemical marker in the sample with the level of the same biochemical marker in a control sample;
wherein a difference in the level of the at least one biochemical marker in the sample relative to the control sample is indicative of an increased risk of developing preeclampsia.

The method according to this disclosure for determining the risk of a pregnant woman developing pre-eclampsia, or having pre-eclampsia, is an *in vitro* method.

This means that the method is carried outside the body of the pregnant woman (also referred to herein as the patient or subject).

In instances where a pregnant individual is determined to have an increased risk of developing pre-eclampsia using a method as described herein, the individual can receive therapy or lifestyle advice from a health care provider. Although there is no widely used treatment for pre-eclampsia, various studies have shown the benefit of therapies such as anti-hypertensive drugs, such as magnesium sulphate, aspirin, diazepam, and phenytoin; and dietary supplements, such as vitamin D, calcium, and selenium.

A sample useful for carrying out the methods described herein generally includes tissues and fluids in which an isoform of PlGF is present. The sample can be any body fluid or tissue sample that contains the selected biochemical markers. Typically a conveniently obtained sample is a body fluid, such as blood and its fractions, such as serum and plasma, sputum or urine obtained from the pregnant woman. It is also possible to use a tissue sample obtained from the pregnant woman, or a cell obtained from the pregnant woman, including a cell which has been placed in or expanded in cell culture. Other exemplary biological samples useful for the methods described herein in amniotic fluid, a chorionic villus biopsy, a placental biopsy and cervicovaginal fluid. Further, samples that have been preserved, such as by freezing or drying (e.g. on blood card format), are suitable for use in the methods described herein. Example 1 describes use of maternal blood in the form of serum. The choice of sample can often depend on the assay formats available in a particular clinical laboratory for testing amounts of markers. For example, some assay formats lack sensitivity needed for assaying whole blood, such that a clinical laboratory opts for testing a fraction of blood, such as serum, or using dried blood.

In certain circumstances, biological samples can be collected on more than one occasion from a pregnant woman, for example, when her hypertensive and/or placental condition requires monitoring for development of pre-eclampsia due to a priori risk, presentation of symptoms and/or other factors. The methods for determining risk of pre-eclampsia described herein can also be used for monitoring a pregnant individual who is undergoing a therapy or treatment for a hypertensive and/or placental condition. If desired, testing of biochemical and/or biophysical markers can be carried out in a home setting, such as by using dipstick biochemical test formats and automated blood pressure machines for home use.

The determination of the PlGF isoforms and other biochemical markers can be carried out during the first trimester or the second trimester of the pregnancy, or during both of the trimesters. However, the ability to detect a high risk of developing pre-eclampsia within the first 12 weeks of pregnancy provides more time for a health care provider to provide prevention strategies for the pregnant woman. It is often desirable to complete a risk assessment early in pregnancy, to allow time for measures for preventing or retarding the PE condition to develop in the woman. Thus, according to one embodiment of the invention the sample is taken during the first trimester of the pregnancy. Typically this means the weeks I to 13 of the pregnancy. According to another embodiment of the invention the sample is taken during the second trimester of the pregnancy. Typically this means the weeks 14 to 26 of the pregnancy.

Pre-eclampsia can develop as early as 20 weeks of gestation and is generally considered "early pre-eclampsia" when it develops before about 32-34 weeks of gestation, and "late pre-eclampsia" when it develops after about 32-34 weeks of gestation. Early pre-eclampsia is associated with increased morbidity and thus is considered a more severe form of pre-eclampsia. The methods for determining the risk ofPE described herein are useful for screening for "early pre-eclampsia" and "late pre-eclampsia." As is described herein, for instance in Example 1, the methods for determining risk of pre-eclampsia are effective during less than 22 weeks of gestation, inclusive, and even less than 13 weeks of gestation. Thus, for use in the methods for detecting pre-eclampsia, a sample can be collected between about 11 and 37 weeks gestation, inclusive, including between about 11 and 20 weeks, inclusive, and more generally, prior to about 20 weeks, within first trimester after about 10 weeks, within second trimester and within third trimester. Although earlier testing is often a beneficial policy from a public health perspective, it is understood that collection of samples can sometimes be affected by practical considerations such as a woman delaying a visit to her health care provider until relatively later weeks of gestation.

The methods described herein can involve, in an embodiment, determining blood pressure of an individual. One or more of systolic blood pressure, diastolic blood pressure and mean arterial blood pressure of the pregnant individual can be used. Mean arterial pressure (MAP) refers to the average blood pressure over a cardiac cycle and is determined by the cardiac output (CO), systemic vascular resistance (SVR), and central venous pressure (CVP), using established procedures. A health care provider can use any method to measure the blood pressure of the pregnant individual, including, for example, palpation methods, auscultatory methods and oscillometric methods. Automated blood pressure measuring equipment also can be used. In an embodiment, the methods described herein also can involve determining uterine artery pulsatility index (PI). By "uterine artery pulsatility index" is meant an arterial blood-flow velocity waveform index designed to quantify the pulsatility or oscillations of the waveform. The PI has been found particularly useful in clinical cases in which there is diastolic flow reversal, i.e. bidirectional flow. The PI of the pregnant individual can be measured using any known method. For example, uterine artery Doppler ultrasonography can be performed via the transvaginal or transabdominal route. The uterine artery is first identified with the use of color Doppler ultrasonography. Pulsed-wave Doppler ultrasonography can then be used to obtain waveforms. Various indices can then be calculated. For example PI can be calculated as the peak systolic flow minus the end diastolic flow divided by the mean flow. Although not wishing to be bound by theory, there is evidence that preeclampsia is a consequence of failure of trophoblastic invasion of the maternal spiral arteries. Doppler ultrasound allows assessing the blood flow pattern in the maternal uterine arteries and identifying pregnancies with impaired trophoblastic invasion.

The methods described herein involve determining the level or amount of a PlGF isoform in a sample. The abbreviation "PlGF" means placental growth factor. PlGF was originally cloned from human term placenta cDNA library in 1991. PlGF has been detected also in other tissues such as heart, lung, muscle and adipose tissue. PlGF belongs to the vascular endothelial growth factor (VEGF) family of proteins. It has a moderate sequence similarity of about 50 % to VEGF-A. Alternative splicing generates four isoforms differing in size of which PlGF-1 and PlGF-2 are believed to be the major isoforms. PlGF-1 (SEQ ID NO:2) contains 131 amino acids (MW MONOMER 14.7 kDa, dimer 29.4 kDa). PlGF-2 (SEQ ID NO:3) contains PlGF-1 and of 21 amino acid heparin binding site insertion (MW monomer 17.3 kDa, dimer 34.6 kDa). The length of the full length PlGF-2 protein is thus 152 amino acids. PlGF-3 (SEQ ID NO:4) contains PlGF-1 and 72 amino acid insertion near the C-terminus (MW = monomer 22.8 kDa, dimer 45.6 kDa). Hence, the length of the full length PlGF-3 protein is 203 amino acids. PlGF-4 (SEQ ID NO:5) contains PlGF-3 and 21 amino acid heparin binding site insertion (MW = monomer 26.2 kDa, dimer 52.4 kDa). The length of the full length PIGF-4 is thus 224 amino acids.

In Fig, 4 is summarized the amino acid sequences of the PlGF Family and relatives. In the precursor SEQ ID NO:1; (Accession no. P49763) amino acids 1 to 18 represent the signal sequence, amino acids 132 to 203 represent a domain of unknown function and in the precursor amino acids 214 to 234 represent the Heparin binding domain. In PlGF-2 (SEQ ID NO:3) amino acids 124 to 144 represent the Heparin binding domain (SEQ ID NO:6). In PlGF-3 (SEQ ID NO:4) amino acids 114 to 185 represent a domain of unknown function (SEQ ID NO:7), referred to herein as "loop 3". In PlGF-4 (SEQ ID NO:5) amino acids 114 to 185 represent the loop 3 and amino acids 196 to 216 the Heparin binding domain.

By "determining the level of one or more biochemical markers in a sample obtained from a pregnant woman, wherein at least one biochemical marker is a PlGF isoform selected from PlGF-2 and PlGF-3" means that a selected isoform is determined by a method specifically assessing the level of the mentioned isoform in a sample obtained from the pregnant woman and from a control sample. The level or amount of a biochemical markers present in a sample can be determined using any assay format suitable for measuring proteins in biological samples. A common assay format for this purpose is the immunoassay, including, for example, enzyme immunoassays (EIA) such as enzyme multiplied immunoassay technique (EMIT), enzyme-linked immunosorbent assay (ELISA), IgM antibody capture ELISA (MAC ELISA), and microparticle enzyme immunoassay (MEIA); capillary electrophoresis immunoassays (CEIA); radioimmunoassays (RIA); immunoradiometric assays (IRMA); fluorescence polarization immunoassays (FPIA); dissociation-enhanced lanthanide fluorescent immunoassay (DELFIA) and chemiluminescence assays (CL).

A method as described herein is exemplified herein by using an isoform specific DELFIA sandwich immunoassay. DELFIA is a heterogenous time-resolved fluorometric assay method that involves measuring a signal from a lanthanide chelate in a protocol employing an enhancement step. The method was used to measure the specific isoforms PlGF-1, PlGF-2 and PlGF-3. The capture antibody in the assay was biotinylated and the detection antibody europium labeled. In these assays can be used the PlGF isoform specific antibodies either from commercial monoclonal antibodies or custom made antibodies. To prepare antibodies that recognize PlGF-3 and do not substantially recognize PlGF-1 or PlGF-2, animals were immunized with antigens comprising peptides containing an amino acid sequence selected from loop 3 (amino acids HSPGRQSPDMPGDFRADAPSFLPPRRSLPMLFRMEWGCALTGSQSAVWPSS PVPEEIPRMHPGRNGKKQQRK; SEQ ID NO:7) of PlGF-3. Specific amino acid sequences that resulted in PlGF-3 selective antibodies (relative to PlGF-2 and PlGF-3) included HSPGRQSPDMPGDFRADA (SEQ ID NO:8) and PEEIPRMHPGRNGKKQQRK (SEQ ID NO:9). Commercially available PlGF-1, PlGF-2 or PlGF-3 proteins can be used as PlGF isoform standards. The measurement can be performed by a time-resolved fluorometer using the factory-set DELFIA protocol.

Typical assay formats for determining the amount of polypeptide and other biomarkers in a sample involve use of a control polypeptide, especially when quantitating levels of amounts of such polypeptides. Commercially available proteins, such as PlGF-1, PlGF-2, PlGF-3 and other biomarkers can be used as standards in assays measuring the level of biochemical markers. Alternatively, methods for expressing proteins, such as in prokaryotic and eukaryotic systems, and for synthesizing polypeptides are well known. Full length proteins and fragments thereof can be used as standards for quantitative determination of levels of biomarkers in a sample obtained from a pregnant woman. Nucleotide sequences of PlGF isoforms are known and published in scientific articles: PlGF-1: Maglione et al. 1991. Isolation of a human placenta cDNA coding for a protein related to the vascular permeability factor; PlGF-2: Hauser et al. A heparin-binding form of placental growth factor (PIGF-2) is expressed in human umbilical vein endothelial cells and in placenta; PlGF-3: Cao et al. (1997) Placental growth factor: identification and characterization of a novel isoform generated by RNA alternative splicing. Such sequences provide guidance for those skilled in the art for expressing PlGF polypeptides for use in methods described herein.

By "a control sample" is here meant a sample obtained from a subject being at the same trimester or gestational age of pregnancy, and wherein the pregnancy is confirmed to have a specific outcome in respect to pre-eclampsia. Typically a "control sample" has been confirmed to have not developed pre-eclampsia (see Examples herein), although use of a control sample confirmed to have developed pre-eclampsia is possible. The term is here defined to encompass one or more samples, so that a control sample can be a set of samples obtained from a population. The pregnant controls chosen can be matched to the pre-eclampsia cases by biophysical parameters, such as maternal age, body mass index, ethnicity and gestational age.

By "an increased risk of developing pre-eclampsia" is meant that the likelihood of the subject (pregnant woman) for developing pre-eclampsia is on a level, which is higher than in a control group of pregnant women not developing PE.

Briefly, an exemplary version of a method as described herein for determining risk of pre-eclampsia of a pregnant woman can be performed by taking a blood sample from the pregnant woman. The blood can be processed to prepare plasma or serum if desired. Assay for a selected subunit of PlGF would be carried out using a standard immunoassay using an antibody selective for a PlGF isoform, such as PlGF-2 or PIGF-3. An example is use of an enzyme linked immunosorbent assay (ELISA) in which intensity of colour development in a test sample is proportional to the concentration of marker present. Based on this test, the level of the PlGF isoform can be calculated. This level can be used in a risk algorithm independently, or in combination with levels of other markers, if desired. To design the risk algorithm, standard logistic regression analysis of a data set adjusted on the assumption of % prevalence of PE in the population can be used. To determine whether the amount of biochemical markers is greater than or less than normal, the normal amount of biochemical marker present in a maternal biological sample from a relevant population is determined. The relevant population can be defined based on any characteristics than can affect normal (unaffected) amounts of the markers. For determining risk of pre-eclampsia, the relevant population can be established on the basis of low risk for pre-eclampsia. Once the normal marker amounts are known, the determined marker amounts can be compared and the significance of the difference determined using standard statistical methods. When there is a statistically significant difference between the determined marker amount and the normal amount, there is a significant risk that the tested individual will develop pre-eclampsia.

The level of the selected biochemical marker in the sample is compared with the level of the same biochemical marker in a control sample. A difference in the level of biochemical marker in the sample relative to the control sample is indicative of an increased risk of developing pre-eclampsia. By a difference is meant a statistically significant difference in the values. By the presence of "increased or decreased levels" of any of the isoforms means that the level of any of the specific isoforms deviates statistically significantly from the level of the same isoform in a control sample being higher or lower than the level in the control sample.

To analyze the measurement results of a single sample in routine screening for pre-eclampsia, data of a control population is first needed. This data is obtained by measuring the selected PlGF isoforms from a large number of samples, preferably more than 100 samples per each week of pregnancy. The measured concentrations of the selected isoforms are then preferably log₁₀ transformed to make the distribution of the biological variation Gaussian. Subsequently, a median concentration and standard deviation for each selected isoform is determined for each pregnancy week from the control data. Afterwards, the results of any single sample can be compared to the appropriate median concentrations to determine whether the concentrations of the selected isoforms differ from their normal values. This comparison can be used as a basis of calculating the patient risk for pre-eclampsia or as a basis of making a diagnosis of pre-eclampsia.

Matched case-control studies can also be made to demonstrate the behaviour of biochemical markers such as the PlGF isoforms. In such studies, a control population that is matched by physiological parameters to the pre-eclampsia case population is used. Consequently, slightly different methods can be used to calculate the results of such study as compared to routine screening. Such a study is exemplified in Example 1.

According to this disclosure a pregnant woman is at increased risk of developing PE, if at least one of the following observations is made:
1. The level of PlGF-2 in the sample relative to the control sample is increased;
2. The level of PlGF-3 is decreased in the subject sample relative to the control sample;
3. The ratio of PlGF-2/PlGF-3 is determined, and a difference in ratio in the subject sample relative to the control sample is observed. Typically, the ratio is increased;
4. The ratio of PlGF-2/PlGF-1 is determined, and a difference in the ratio in the subject sample relative to the control sample is observed. Typically, the ratio is increased. The increased ratio is observed in particular during the second trimester of pregnancy.

The method as described herein can be combined with the determination of one or more biochemical markers is selected from PAPP-A, PAI-1, PAI-2, PlGF-1, PIGF-4, ADAM-12, PP13, and VEGF165b.

As used herein, the term "PAPP-A" means the metzincin metalloproteinase known as Pregnancy-associated plasma protein A and having an amino acid sequence homologous to GenBank accession number AAH78657. As used herein, the term "PP13" means placental protein 13, also known as galectin 13 having an amino acid sequence homologous to GenBank accession number NP_037400. As used herein, the term "PAI-1" means Plasminogen activator inhibitor 1, also known as PAI and Endothelial plasminogen activator inhibitor, and having an amino acid sequence homologous to UniProt accession number P05121. As used herein, the term "PAI-2" means Plasminogen activator inhibitor 2, also known as Placental plasminogen activator inhibitor, Monocyte Arg-serpin and Urokinase inhibitor, and having an amino acid sequence homologous to UniProt accession number P05120. As used herein, the term ADAM-12 means Disintegrin and metalloproteinase domain-containing protein 12, also known as Meltrin-alpha, and having an amino acid sequence homologous to UniProt accession number 043184, As used herein, the term "VEGF 165b" means vascular endothelial growth factor splice variant 165b and having an amino acid sequence homologous to UniProt accession number P15692-8.

When the one or more biochemical markers are compared with the same biochemical marker in a control subject, an increased or decreased measure of the biochemical marker in the pregnant woman relative to the control is indicative of an increased risk of developing pre-eclampsia.

The method as described herein can be combined also with the determination of one or more biophysical markers. The biophysical marker can be selected from blood pressure and uterine artery pulsatility index.

When the one or more biophysical markers are compared with the same biophysical marker in a control subject, an increased or decreased measure of the biophysical marker in the pregnant woman relative to the control is indicative of an increased risk of developing pre-eclampsia.

The detection method as described herein can be used also for diagnosing pre-eclampsia. Confirmation that a pregnant woman has pre-eclampsia can be typically carried out during second or third trimester of pregnancy, such as the 20th to 40th weeks of pregnancy; in some cases even before week 20.

According to one further embodiment a method for determining whether a pregnant woman has pre-eclampsia, comprises:
i) Determining the level of one or more biochemical markers in a sample obtained from a pregnant woman, wherein at least one biochemical marker is a PlGF isoform selected from PlGF-2 and PlGF-3;
ii) Comparing the level of the at least one biochemical marker in the sample with the level of the same biochemical marker in a control sample;
wherein a difference in the level of the at least one biochemical marker in the subject sample relative to the control sample is indicative of pre-eclampsia.

According to this disclosure a pregnant woman has PE, if at least one of the following observations is made:
1. The level of PIGF-2 in a sample obtained from a subject is increased relative to the level of PIGF-2 in the control sample;
2. The level of PIGF-3 in a sample obtained from a subject is decreased relative to the level of PIGF-3 in the control sample;
3. A difference in ratio of PIGF-2/PIGF-1 in a sample obtained from a subject relative to the control sample is observed;
4. A difference in the ratio of PIGF-2/PIGF-3 in a sample obtained from a subject relative to the control sample is observed.

Typically, both the ratio of PIGF-2/PIGF-1 and the ratio of PIGF-2/PIGF-3 are increased in a sample obtained from a subject relative to the control sample.

The determination whether a pregnant woman has PE is carried out during weeks 20 - 40 of pregnancy.

The method for determining whether a pregnant woman has pre-eclampsia can be combined with one or more biochemical markers selected from PAPP-A, PAI-1, PAI-2, PP13, VEGF165b, PIGF-1 and PIGF-4.

The method as described herein can be combined also with the determination of one or more biophysical markers, such as blood pressure and/or uterine artery pulsatility index.

According to one further embodiment a computer program which when executed on a computer causes the computer to perform a process for determining whether a pregnant woman is at risk of developing pre-eclampsia or determining the presence of the condition, wherein the process comprises:
inputting a measurement of at least one biochemical marker obtained by:
   1) assaying a sample obtained from the pregnant woman for one or more biochemical markers, wherein at least one biochemical marker is selected from PIGF-2 and PIGF-3;
ii) comparing the level of the one or more biochemical marker in the sample with the level of the same biochemical marker in a control sample, wherein a difference in level of the one or more biochemical marker in the sample relative to the control sample is indicative of pre-eclampsia, and
iii) determining a quantitative estimate of pre-eclampsia risk based on the result of the comparing.

The computer program can further comprise assaying for at least one biochemical marker selected from the group comprising PAPP-A, PAI-1, PAI-2, ADAM-12, PP13, VEGF165b, PIG-1 and PIGF-4.

The computer program can further comprise inputting a measurement of at least one biomarker obtained by determining one or more biophysical markers of the subject; comparing the one or more of the one or more biophysical markers of the subject with the same biophysical marker in a control subject, wherein an increased or decreased measure of the one or more one or more biophysical marker in the subject relative to the control is indicative of an increased risk of developingpre-eclampsia. Determining a quantitative estimate of pre-eclampsia risk based on the result of the compared one of more biochemical marker and the compared one or more biophysical marker.

The computer program can further comprise the use of a biophysical marker selected from blood pressure and uterine artery pulsatility index.

The determination of the quantitative estimate of pre-eclampsia risk comprises determining the likelihood of pre-eclampsia using a multivariate analysis. The multivariate analysis comprises using levels of the biochemical markers and distribution parameters derived from a set of control reference data. The multivariate analysis is a preferably a multivariate Gaussian analysis.

The description relates also to a computer program recording medium stoting the computer program as described herein.

The present invention provides a kit for assessing risk of developing pre-eclampsia or the presence of pre-eclampsia in a pregnant woman. In an embodiment, the kit can include i) at least two detectable binding partners, wherein each detectable binding partner binds specifically to an individual PIGF isoform selected from PIGF-1, PIGF-2 and PIGF-3. In another embodiment, the kit can include i) a detectable binding partner that binds specifically to PIGF-2, and ii) a detectable binding partner that binds specifically to a PIGF isoform selected from PIGF-1 and PIGF-3. In a further embodiment the kit can include i) a detectable binding partner that binds specifically to PIGF-3, and ii) a detectable binding partner that binds specifically to a PIGF isoform selected from PIGF-1 and PIGF-2. A detectable binding partner used in a kit as described herein can be, for example, an antibody or antigen-binding fragment thereof. Other binding partners useful for detecting protein-protein interactions also can be used.

The present invention provides also a kit for assessing risk of developing pre-eclampsia or the presence of pre-eclampsia in a pregnant woman. The kit can include i) at least one antibody or an antigen-binding fragment thereof, that binds specifically to a PIGF isoform selected from PIGF-2 and PIGF-3; and ii) instructions for using the antibody or antigen-binding fragment in the determination.

By instructions is meant guidelines how to carry out the determination by using the antibodies or antigen-binding fragments Reagent volumes, incubation times, reaction conditions etc. can be provided in the instructions.

The antibody or antigen-binding fragment thereof can bind to a specific structure of an isoform, which can be a linear sequence of amino acids, a folded polypeptide aminio acid sequence, and any post-translational modification therein. In an embodiment, an antibody or antigen-binding fragment selective for PIGF-2 can binds to the sequence between amino acids 124 to 144 in mature PIGF-2 (SEQ ID NO:6). In an embodiment, an antibody or antigen-binding fragment selective for PIGF-3 can bind to the sequence between amino acids 114 to 185 in mature PIGF-3 (SEQ ID NO:7).

The methods described herein involve selectively detecting an isoform of PIGF using a laboratory test procedure. Such a test would employ an agent capable of selectively detecting an isoform of PIGF. Such an agent would not appreciably detect non-targeted PIGF isoform, intact PIGF and other molecules. An exemplary agent capable of selectively detecting an isoform of PIGF is an antibody selective for one of PIGF-1, PIGF-2, PIGF-3, PIGF-4 or another PIGF isoform. The assessment of the level of a selected isoform can be carried out for example by using an immunological method, where antibodies specifically bind to the selected isoform. An antibody can be selectively raised against the whole isoform and those antibodies that are specific against a specific structure of the isoform, such as loop 3 structure, are selected. Alternatively, antibodies can be raised against a specific structure of the isoform, such as loop 3 structure, of each isoform. Methods for preparing and characterizing antibodies are well known in the art (See, e.g. Harlow and Lane, 1988 Harlow, E. & Lane, D. Antibodies: A Laboratory Manual. Cold Spring Harbor Laboratory Press, New York, 1988, page 14 (incorporated herein by reference). The methods for generating mAbs generally begin along the same lines as those for preparing polyclonal antibodies. Briefly, a polyclonal antibody is prepared by immunizing an animal with one or more PIGF protein isoforms and collecting antisera from that immunized animal. A wide range of animal species can be used for the production of antisera. Typically the animal used for production of anti-antisera is rabbit, a mouse, a rat, a hamster, a guinea pig or a goat. MAbs can be readily prepared through use of well-known techniques, such as those exemplified in U.S. Pat. No. 4,196,265, incorporated herein by reference. Typically, the technique involves immunizing a suitable animal with a selected immunogen composition. The immunizing composition is administered in a manner effective to stimulate antibody producing cells. Rodents such as mice and rats are commonly used animals, however, the use of rabbit, sheep, or frog cells is also possible. Described herein are particular PLGF isoform-specific antibodies capable of distinguishing specific isoforms from each other.

The risk that a pregnant individual develops pre-eclampsia can be determined from biochemical marker amounts using statistical analysis based on clinical data collected in a patient population study. There are multiple statistical methods for combining parameters that characterize the pregnant individual, such as amounts of biochemical markers, to obtain a risk estimate. The likelihood method (Palomaki and Haddow, Am. J. Obstet. Gynecol. 156, 460-3 (1987)) and the linear discriminant function method (Norgarrd-Pedersen et al. Clin. Genet. 37, 35-43 (1990)) are commonly used for this purpose. As such, the methods described herein for determining risk can be based on use of well known statistical methods, in which a cutoff or a MoM is used to determine risk.

To outline an exemplary approach to risk determination, the basic principle of the likelihood method is that the population distributions for a parameter (such as the amount of a biochemical marker) are known for the 'unaffected' and 'affected' groups. Thus, for any given parameter (such as amount of marker and blood pressure reading), the likelihood of membership of the 'unaffected' and 'affected' groups can be calculated. The likelihood is calculated as the Gaussian height for the parameter based on the population mean and standard deviation. The 'likelihood ratio' is the ratio of the heights calculated using 'unaffected' and 'affected' population parameters, and is an expression of the increased risk of having a disorder, with respect to a prior risk. A woman's prior odds (which is a statistical expression related to prior risk, as is described herein below) for having pre-eclampsia or carrying a fetus with a chromosomal abnormality can be calculated using a formula derived by clinical population studies using known methods. These prior odds can be modified using the likelihood ratio to derive the posterior odds that can be used for the pre-eclampsia risk estimate. A detailed description of use of the likelihood method for predicting risk that a fetus has a chromosomal abnormality can be applied to predicting risk of pre-eclampsia and is set forth, for example, in "Screening for Down's Syndrome," ed. J.G Grudzinskas, T. Chard, M. Chapman and H. Cuckle; Published by Cambridge University Press, 1994). It is also possible to use observed distributions of likelihood ratios for determining risk using the methods described herein (see, for example, Spencer et al. Ann. Clin. Biochem., 29, 506-18(1992)).

As an example of an approach for determining a risk that a pregnant woman develops pre-eclampsia, samples can be collected from a population of women known to have had pre-eclampsia. These samples are analyzed to determine the level of one or more PIGF isoforms. The determined level of PIGF isoform(s) would typically then be converted to a multiple of the expected normal median (MoM) specific to a pregnancy of the same gestational age, maternal weight, ethnicity, smoking status, method of conception and parity. Well known statistical regression approaches would then be used for risk calculations (see, for example Draper et al. Applied Regression Analysis (3th ed.) Wiley: New York, NY, 1998 and Cuckle HS et al. , Estimating a woman's risk of having a pregnancy associated with Down's syndrome using her age and serum alphafetoprotein level. Br. J Obstet Gynacol 1987; 94:387-402; and other references above).

Previous reports have indicated that the level of PIGF in maternal blood can be used as a predictor of Down syndrome. It is therefore expected that detection of one or more specific isoforms of PIGF in a maternal sample can be used for detecting fetal chromosomal abnormalities.

### Examples

### Example 1

This example describes that the level of PIGF-2 in maternal serum is increased in subjects who develop pre-eclampsia while the level of PIGF-3 in maternal serum is decreased in subjects who develop pre-eclampsia.

PIGF-isoform specific DELFIA sandwich assays were developed for measuring (a) PIGF-2; (b) PIGF-3 and (c) the combination of PIGF-1, PIGF-2 and PIGF-3.

PIGF isoforms were measured in serum obtained from pregnant women who subsequently developed pre-eclampsia and pregnant women unaffected by pre-eclampsia. Two blood samples were drawn from each woman: one during 1^{st} trimester and the second during 2^{nd} trimester of pregnancy. The blood tubes were centrifuged and serum was collected and aliquoted. These aliquots were stored at - 20°C. The unaffected pregnancy controls chosen were matched to the pre-eclampsia pregnancy cases by biophysical parameters such as maternal age, body mass index, ethnicity and gestational age. PIGF-2 and PIGF-3 concentrations were measured in separate assays from eight different pre-eclampsia case samples and 16 matched control samples. The statistical analysis was done from these results.

In these assays the PIGF isoform specific antibodies were either commercial monoclonal antibodies or custom made antibodies. The capture antibody in the assay was biotinylated and the detection antibody was labeled with DELFIA europium chelate. 0.2 µg of biotinylated isoform specific antibody in 200 µl volume was incubated in streptavidin coated microtitration plates at 25 °C for 30 min. After washing, the 100 µl of serum pregnancy sample orPIGF standard was added and incubated for 2 hours. PIGF standards were commercial PIGF-1, PIGF-2 or PIGF-3 proteins. Wells were washed again and 0.2 µg of the Eu-anti-PIGF in 200 µl volume was added and incubated for 1 hour. Enhancement solution was added after wells were washed to develop the Eu signal. The measurement s was performed by a time-resolved fluorometer (Victor 2) at 615 nm using the factory-set DELFIA Eu protocol.

Fig. 1 shows the concentration of (a) PIGF-2 as "X"; (b) PIGF-3 as squares; and (c) PIGF-1, PIGF-2 and PIGF-3 as triangles. The concentration (pg/ml serum) PIGF isoform measured in each case increases as pregnancy progresses in women unaffected by pre-eclampsia, until about week 30, after which the concentrations eventually start to decline.

Fig. 2 describes the results of a matched case-control study in which samples taken from pregnant, non-pre-eclamptic females (controls, represented by triangles in the illustration) and samples taken from pregnant females that later on in their pregnancy developed pre-eclampsia (cases, represented by crosses in the illustration) were measured with a method that detects PIGF-2 isoform. The controls chosen were matched to the cases by their biophysical parameters (maternal age, body mass index, ethnicity and gestational age). Visual inspection of the results indicates that the concentrations measured from the case samples were elevated as compared to the control samples during both the 1^{st} and the 2^{nd} trimester of pregnancy.

Fig. 3 describes the results of a matched case-control study in which samples taken from pregnant, non-pre-eclamptic females (controls, represented by triangles in the illustration) and samples taken from pregnant females that later on in their pregnancy developed pre-eclampsia (cases, represented by crosses in the illustration) were measured with a method that detects PIGF-3 isoform. The controls chosen were matched to the cases by their biophysical parameters (maternal age, body mass index, ethnicity and gestational age). Visual inspection of the results indicates that the concentrations measured from the case samples were essentially the same as compared to the control samples during the 1^{st} trimester of pregnancy, but clearly lower as compared to the control samples during the 2^{nd} trimester of pregnancy.

When analyzing the results of the matched case-control study, the measured concentrations of PIGF-2 and PIGF-3 isoforms were log₁₀ transformed to make the distribution of the biological variation Gaussian. Then, the medians, averages and standard deviations of the log₁₀ concentrations of the PIGF-2 and PIGF-3 isoforms in the samples of the pre-eclampsia case and control populations were calculated separately for the 1^{st} (gestational age 1-13 weeks) and 2^{nd} (gestational age 14-26 weeks) trimesters. Finally it was calculated how many multiples of the standard deviation of the control population the median of the pre-eclampsia case population differed from the median of the control population. The results of these calculations are summarized in Tables 1A (PIGF-2) and 1B (PIGF-3).

**Table 1A**

| | Log₁₀ of PIGF-2 Concentration | | | | | | | |
|---|---|---|---|---|---|---|---|---|
| | 1st trimester of pregnancy (weeks 1-13) | | | | 2nd trimester of pregnancy (weeks 14-26) | | | |
| | Median | Average | SD | Difference (xSD of controls) | Median | Average | SD | Difference (xSD of controls) |
| Controls | 1.86 | 1.92 | 0.447 | | 2.23 | 2.19 | 0.247 | |
| PE | 2.20 | 2.25 | 0.360 | -0.761 | 2.42 | 2.37 | 0.236 | 0.769 |

**Table 1B**

| | Log₁₀ of PIGF-3 Concentration | | | | | | | |
|---|---|---|---|---|---|---|---|---|
| | 1st trimester of pregnancy (weeks 1-13) | | | | 2nd trimester of pregnancy (weeks 14-26) | | | |
| | Median | Average | SD | Difference (xSD of controls) | Median | Average | SD | Difference (xSD of controls) |
| Controls | 1.98 | 1.90 | 0.263 | | 2.21 | 2.20 | 0.178 | |
| PE | 1.95 | 1.90 | 0.245 | 0.114 | 2.12 | 2.01 | 0.321 | 0.506 |

The results showed that the median concentration of PIGF-2 isoform in the samples from the pre-eclampsia population was higher than in the samples from the control population during both the 1^{st} and the 2^{nd} trimesters of pregnancy. This difference in medians in both trimesters was about 0.8 times the standard deviation of the control population results and it was concluded that the concentration of PIGF-2 isoform in blood would be a more useful measurand when predicting the risk of an individual for developing pre-eclampsia later on in pregnancy, as has been done before with other measurands (Akolekar et al. (2008)).

The median concentration of PIGF-3 isoform in the samples from the pre-eclampsia population was lower than in the samples from the control population during the 2^{nd} trimester of pregnancy. This difference in medians was about 0.5 times the standard deviation of the control population results. The difference of medians between the pre-eclampsia and control populations during the 1^{st} trimester of pregnancy was too small to be significant. It was concluded that the concentration of PIGF-3 isoform in blood during the 2^{nd} trimester is a useful measurand when predicting the risk of an individual for developing pre-eclampsia later on in pregnancy.

### Example 2

This method shows that a commercially available assay for PIGF-1 has cross-reactivity with other PIGF isoforms.

PIGF-1 was assayed using a commercial DELFIA Xpress PIGF method (PerkinElmer). Samples were prepared to contain known amounts of purified recombinant PIGF isoforms, including recombinant PIGF-1 (non-glycosylated). It was observed as expected that the PIGF-1 antibody provided with the DELFIA Xpress kit was highest with PIGF-1 (Table 2). However, significant cross-reactivity to the PIGF-2 isoform and some cross-reactivity to the PIGF-3 isofom were also observed. Thus this method mainly detects PIGF-1, but not specifically.

Similar results have been observed by other manufacturers with their current PIGF-1 methods. For example, R&D Systems reports in their method instructions a 50% cross-reactivity with PIGF-2 as measured against standards prepared from PIGF-1 by their Quantikine Human PIGF ELISA kit. Roche reports in their method instructions a 28% cross-reactivity with PIGF-2 as measured against standards prepared from PIGF-1 by their Elecsys PIGF assay.

**Table 2. DELFIA Xpress PIGF Assay cross-reactivity:**

| **Tested substance** | **Tested concentration (pg/mL)** | **Observed cross-reactivity (%)** |
|---|---|---|
| PIGF-1 (glycosylated) | **5000** | **33** |
| PIGF-2 (glycosylated) | **5000** | **16** |
| PIGF-3 (non-glycosylated) | **5000** | **5** |

## Claims

1. A kit for assessing risk or presence of pre-eclampsia in a pregnant woman, comprising
i) at least two detectable binding partners, wherein each detectable binding partner binds specifically to an individual PIGF isoform selected from PIGF-1, PIGF-2 and PIGF-3.

2. The kit according to claim 1, comprising:
i) a detectable binding partner that binds specifically to PIGF-2, and
ii) a detectable binding partner that binds specifically to a PIGF isoform selected from PIGF-1 and PIGF-3.

3. The kit according to claim 1, comprising:
i) a detectable binding partner that binds specifically to PIGF-3, and
ii) a detectable binding partner that binds specifically to a PIGF isoform selected from PIGF-1 and PIGF-2.

4. The kit according to any one of claims 1 to 3, wherein the detectable binding partner is an antibody or antigen-binding fragment thereof.

5. The kit according to claim 4 , wherein the antibody or antigen-binding fragment thereof binds to the sequence between amino acids 124 to 144 in mature PIGF-2 (SEQ ID NO:6) or to the sequence between amino acids 114 to 185 in mature PIGF-3 (SEQ ID NO:7).

6. The kit according to claim 5, wherein the antibody or antigen-binding fragment thereof binds to a PIGF-3 sequence selected from HSPGRQSPDMPGDFRADA (SEQ ID NO:8) and PEEIPRMHPGRNGKKQQRK (SEQ ID NO:9).

## Patentansprüche

1. Kit zum Bewerten des Risikos oder des Vorhandenseins einer Präeklampsie bei einer schwangeren Frau, der Folgendes umfasst:
i) mindestens zwei nachweisbare Bindungspartner, wobei jeder nachweisbare Bindungspartner in spezifischer Weise an ein individuelles PIGF Isoform bindet, das ausgewählt wird aus PIGF-1, PIGF-2 und PIGF-3.

2. Kit nach Anspruch 1, umfassend:
i) einen nachweisbaren Bindungspartner, der in spezifischer Weise an PIGF-2 bindet und
ii) einen nachweisbaren Bindungspartner, der in spezifischer Weise an ein PIGF Isoform bindet, das ausgewählt ist aus PIGF-1 und PIGF-3.

3. Kit nach Anspruch 1, umfassend:
i) einen nachweisbaren Bindungspartner, der in spezifischer Weise an PIGF-3 bindet und
ii) einen nachweisbaren Bindungspartner, der in spezifischer Weise an ein PIGF Isoform bindet, das ausgewählt ist aus PIGF-1 und PIGF-2.

4. Kit nach einem der Ansprüche 1 bis 3, wobei der nachweisbare Bindungspartner ein Antikörper oder ein Antigen-bindendes Fragment davon ist.

5. Kit nach Anspruch 4, wobei der Antikörper oder das Antigen-bindende Fragment davon an die Sequenz zwischen den Aminosäuren 124 bis 144 in reifem PIGF-2 (SEQ ID NO:6) oder an die Sequenz zwischen den Aminosäuren 114 bis 185 in reifem PIGF-3 (SEQ ID NO:7) bindet.

6. Kit nach Anspruch 5, wobei der Antikörper oder das Antigen-bindende Fragment davon an eine PIGF-3 Sequenz bindet, die ausgewählt wird aus HSPGRQSPDMPGDFRADA (SEQ ID NO:8) und PEEIPRMHPGRNGKKQQRK (SEQ ID NO:9).

## Revendications

1. Kit destiné à évaluer le risque de présence d'une pré-éclampsie chez une femme enceinte, comprenant :
i) au moins deux partenaires de liaison détectables, dans lequel chaque partenaire de liaison détectable se lie spécifiquement à une isoforme de PIGF individuelle sélectionnée parmi PIGF-1, PIGF-2 et PIGF-3.

2. Kit selon la revendication 1, comprenant :
i) un partenaire de liaison détectable qui se lie spécifiquement à PIGF-2, et
ii) un partenaire de liaison détectable qui se lie spécifiquement à une isoforme de PIGF sélectionnée parmi PIGF-1 et PIGF-3.

3. Kit selon la revendication 1, comprenant :
i) un partenaire de liaison détectable qui se lie spécifiquement au PIGF-3, et
ii) un partenaire de liaison détectable qui se lie spécifiquement à une isoforme de PIGF sélectionnée parmi PIGF-1 et PIGF-2.

4. Kit selon l'une quelconque des revendications 1 à 3, dans lequel le partenaire de liaison détectable est un anticorps ou un fragment de celui-ci se liant à un antigène.

5. Kit selon la revendication 4, dans lequel l'anticorps ou le fragment de celui-ci se liant à un antigène se lie à la séquence entre les acides aminés 124 à 144 dans PIGF-2 mature (SEQ ID NO : 6) ou à la séquence entre les acides aminés 114 à 185 dans PIGF-3 mature (SEQ ID NO : 7).

6. Kit selon la revendication 5, dans lequel l'anticorps ou le fragment de celui-ci se liant à un antigène se lie à une séquence de PIGF-3 sélectionnée parmi HSPGRQSPDMPGDFRADA (SEQ ID NO : 8) et PEEIPRMHPGRNGKKQQRK (SEQ ID NO : 9).
